# EUROPEAN PATENT SPECIFICATION

(11) **EP 3 364 988 B1**
(45) Date of publication and mention of the grant of the patent: **28.07.2021**
(21) Application number: 16820036.8
(22) Date of filing: 19.10.2016
(51) Int. Cl.: A61K 35/644, A61K 36/30, A61K 36/63, A61P 17/02

(54) **WOUND CARE COMPOSITION**
WUNDPFLEGEZUSAMMENSETZUNG
COMPOSITION DE SOIN DES PLAIES

(30) Priority: 22.10.2015 TR 201513210
(43) Date of publication of application: 29.08.2018
(73) Proprietor: Karaman Ozlu, Zeynep, Erzurum (TR); Gumus, Kenan, Amasya (TR); Atatürk Üniversitesi Bilimsel Arastirma Projeleri Birimi, Merkez Erzurum (TR)
(72) Inventor: KARAMAN OZLU, Zeynep, Erzurum (TR); GUMUS, Kenan, Erzurum (TR)
(74) Representative: Yamankaradeniz, Kemal
(86) International application number: PCT/TR2016/050388
(87) International publication number: WO 2017/069715

(56) References cited:
- EP-A1- 2 364 713
- WO-A1-01/21212
- GB-A- 190 508 353
- US-A- 4 282 250
- US-A1- 2003 082 130
- US-A1- 2005 112 208
- A ASSIMOPOULOU: "Antioxidant activities of alkannin, shikonin and Alkanna tinctoria root extracts in oil substrates", FOOD CHEMISTRY, vol. 87, no. 3, 1 September 2004 (2004-09-01), pages 433-438, XP055343170, NL ISSN: 0308-8146, DOI: 10.1016/j.foodchem.2003.12.017
- V. PAPAGEORGIOU ET AL: "Alkannins and Shikonins: A New Class of Wound Healing Agents", CURRENT MEDICINAL CHEMISTRY : THE NEW INTERNATIONAL JOURNAL FOR TIMELY IN-DEPTH REVIEWS IN MEDICINAL CHEMISTRY, vol. 15, no. 30, 1 December 2008 (2008-12-01), pages 3248-3267, XP055343161, NL ISSN: 0929-8673, DOI: 10.2174/092986708786848532
- GÜMÜS KENAN ET AL: "The effect of a beeswax, olive oil andAlkanna tinctoria(L.) Tausch mixture on burn injuries: An experimental study with a control group", COMPLEMENTARY THERAPIES IN MEDICINE, vol. 34, 10 August 2017 (2017-08-10), pages 66-73, XP085202234, ISSN: 0965-2299, DOI: 10.1016/J.CTIM.2017.08.001

## Description

### The Related Art

The invention relates to a wound care composition for dressing and effective treatment of burn wounds in burn units and burn centers.

The invention particularly relates to a wound care composition for use in treatment of 2^{nd} degree burns, preventing sticking of dressing material to wounded tissue during dressing, reducing the pain caused by the dressing during wound care, speeding up the wound treatment process, and protecting the wound against infections.

### The Prior Art

Wound is any damage that a physical or chemical factor causes in a way to disrupt body's integrity. Temporary or permanent disappearance of physiological properties of tissues upon the disruption, destruction of the integrity of tissue or mucous membrane, cutting of tissues as a result of physical or chemical factors is called wound.

Wounds are classified as open or closed wounds. Burn wounds are also fallen under the category of open wounds. Burn wounds are those that occur locally as a result of exposure to heat, flame, toxic materials or radioactive rays.

The way of treatment is very important in burn wounds. During dressing, sterile sponge or pads should not be stuck to the wounded tissue. However wound care materials (sterile sponge or pad or gauze) that are used today, stick to the wound site during dressing change, traumatizing the wound. This prolongs the recovery period and accordingly patient's stay in the hospital, also increases the pain felt by the patient during dressing change. The reason for this is the content of wound or burn ointments that are used.

During dressing procedures, in which the prior art wound or burn ointments are used, a minimum deviation from the aseptic principles results in wound site infection. Aseptic principles can be summarized as follows: pathogen-free environment; cleaning up the dressing room after each dressing; use of sterile materials; using new dressing kit for every patient; using sterile gloves, masks and aprons; and use of antiseptic solutions. Maintaining these principles which prevents wound infection in patients during wound care is of optimum importance. In case of a minimum deviation from the aseptic principles, the person is dealing both with the burn wound and clearing the infection; the care period of the burn wound -which becomes a much more challenging state after an infection- follows a more complex prognosis. This also wears the person out psychologically.

Moreover the available wound ointments that are used causes nociceptive effect such burning, aching and pain during the application on wounded tissue, and this is a very difficult thing considering it causes more pain in a patient who is already in pain due to the wounded tissue.

Furthermore the products that are described to be effective in available wound care materials and compositions are quite expensive and cause an extra cost for hospitals. Thus, some of the healthcare organizations prefer the cheaper open wound care products whose effectiveness are questionable.

Additionally as described in the literature, with the use of traditional wound and burn ointments it takes a rather long period of an average 3 weeks for a 2^{nd} degree burns to heal. In other words, recovery period is substantially long. This prevents the person from doing his daily activities and self care.

As one of the inventions in the literature, "Vacuum Treatment and Cleansing Dressing for Wounds" titled invention with the Application No. EP01998292.5 and class of A61F 13/00 IPC, comprises a bandage for use with a vacuum source, a component to be placed in contact with the wound surface and a suction port in accordance with the component. The component is configured with passageways and/or spacers.

As another application in the literature, "A Dressing Product" titled invention having the Application Number of EP06727400.1 relates to a dressing product. The dressing product includes the first and second absorbing layers in the form of a nonwoven fabric made up of viscose and polyester. Each absorbing layer has a working inner surface and a working outer surface. First and second absorbing layers are joined together with their working inner surfaces are facing each other thus these absorbing layers together form a tampon. Connection between the first and second absorbing layers are made through a needling process with the density of needling is 400 punch/cm2 at the most. The dressing product also includes a third layer that is placed as a sandwich between the first and second layers, joining with them. The third layer is cotton gauze. The scope of the invention also extends in a way to include a method for the making of the dressing product and a method for treating a wound.

Another application encountered in the literature is "Burn and wound ointment" titled invention with the application No. TR 2012 00429. This invention relates to a composition of olive oil, red apple and white wax that is used in the treatment of skin burns and wounds and provides a faster, scarless recovery.

Another application in the literature is, "Process for the preparation of ozone and peroxide derivatives of fatty acids or their esters in the form of a spreadable ointment" titled invention, with the Application No. EP02023866.3. This invention, a process for the preparation of ozone and peroxide derivatives of fatty acids or their esters in the form of a spreadable ointment that is easy to use in a topical way, provides the mixing of ozonides and peroxides with a commercially available ready-to-use ointment base which is made of paraffin oil thickened with polyethylene; these paraffin oils are ineffective against ozonides and peroxides as much as possible, thus the ointment maintains a high degree physical strength without phase separation and colour change.

After the applications are examined, it was seen that no embodiment was developed for preventing sterile sponge, pad, and gauze etc. dressing materials from sticking to the wounded or burnt tissue.

As a result, improvement is to be made in wound care compositions, and therefore novel embodiments that would eliminate the above said drawbacks and bring solutions to the problems of the prior art systems are needed.

### Purpose of the Invention

The invention relates to a wound care composition and a method of obtaining the wound care composition, which meets the above said requirements, eliminates all of the drawbacks, and brings some additional advantages.

The primary purpose of the invention is to prevent the potential wound-site infections that could occur during wound care applications. In order to ensure this, olive oil, beeswax, and alkanna tinctoria is used in the wound care composition. Because each of these basic materials is an antioxidant, anti-inflammatory and anti-bacterial, they prevent infections.

Another purpose of the invention is to prevent ache and pain induced by wound ointment during wound dressing. This is provided by the nociceptive effect of the alkanna tinctoria.

Another purpose of the invention is to prevent the dressing materials from sticking on the wounded tissue during dressing by removing the dressing materials such as gauze, sponge or pads from the wounded tissue. And this is achieved with olive oil. This way, during the dressing that is done with the wound care composition, it is provided that dressing material does not stick to the wound and therefore does traumatize the wound. Also with this way, it is provided that dressing changes are done in a less painful way.

Another purpose of the invention is to shorten wound recovery period. This is provided with the alkanna tinctoria's contribution to the vascularisation of the wound. For better understanding of the embodiment of the present invention and its advantages with its additional components, it should be evaluated together with below described figures.

### DETAILED DESCRIPTION OF THE INVENTION

In this detailed description, the preferred embodiments of the invention are only disclosed for better understanding of the subject.

The invention generally relates to a wound care composition basically consisting beeswax, olive oil, and alkanna tinctoria, for use in treatment of wounds, particularly 2^{nd} degree burn wounds, preventing the wound care material from sticking to the wounded tissue during dressing, reducing the wound treatment time period, and preventing formation of infection at the wounded surface.

*In the below given table, the usable amounts by weight percent and the preferred percentages of said components are given:*

| Component Name | Preferred amount by weight (%) | Usable amount by weight (%) |
|---|---|---|
| Beeswax | 2.78 | 2.50- 12.00 |
| Olive Oil | 92.59 | 92.00 - 96.00 |
| Alkanna Tinctoria | 4.63 | 3.00 - 12.00 |

Beeswax, olive oil, and alkanna tinctoria form the characteristic of the invention.

### Raw Material Properties of the Invention:

### 1. Beeswax:

Beeswax is an anti-oxidant, anti-bacterial material, which has an effect of increasing the production of cytokine in the skin cells and an effect of reducing exudates on the wound site. In addition to these effects, the beeswax that is added to the composition has a function of transforming the composition into a gel form applicable on the wound. In the literature it is stated that the purified, cleaned beeswax is used in the pharmaceutical industry and in the production of creams. When applied on the living tissue, it does not have an allergic or toxic effect.

### 2. Olive Oil:

Besides its bactericidal effect such as hydroxytyrosol and oleuropein, olive oil is a material that has at least 30 phenolic components. As a metabolite of oleuropein, hydroxytyrosol is effective on Haemophilus influenzae, Moraxella catarrhalis, Salmonella typhi, Vibrio parahaemolticus and S. areus. It is also particularly effective on Klebsiella and Pseudomonas bacteria types that are resistant against antibiotics. Oleuropein shows a strong anti-microbial function. Phenolic acid inside the olive oil has an effect of protecting the cell against the cytotoxic effect of the reactive oxygen. In addition to this, oil olive which is richer than the flavonoids, is an anti-inflammatory that inhibits the secretion of IL6, IL8 and histamine, an anti-oxidant, and an anti-oxidant that has a cell protection effect. And this provides the protection of the wounded tissue against the infections.

The acid ratio of the olive oil that is used during the wound care composition of the invention is less than 0.8 %. Thus, a medical olive oil with an acid ratio less than 0.8 % is used while creating a wound care composition. Medical olive oil generally has similar properties compared to other pure olive oils in terms of chemical properties. However for an olive oil to be described as a medical olive oil, its acid ratio is of significant importance. And as described above, this ratio has to be less than 0.8 %.

Through adding olive oil to the composition, it is provided that beeswax melts and the essence of alkanna tinctoria reflects in the composition.

### 3. Alkanna Tinctoria:

Alkannin and shikonin, which are obtained from the roots alkanna tinctoria plant that is colloquially known as dyer's alkanet, are derivatives and active substances of this plant. Shikonin is a red coloured naphthoquinone pigment that is produced from the cells of the plant. Alkannin is the first pigment substance that is isolated from root of the dyer's alkanet plant and considered as the primary element of the red coloured pigments. They are used in pharmaceutical industry, cosmetics and the coloration of liquors. Alkanna tinctoria is a substance that has an anti-microbial effect against staphylococcus aureus and staphylococcus epidermitis. And this provides the protection of the wounded tissue against the infections.

In addition to the positive effects on wound healing, it is said described to have a non-toxic, anti-tumour, anti-thrombolytic effects. Alkanna tinctoria, which has a widespread use in the traditional medicine field in China, is one the most recommended anti-septic and anti-inflammatory ointments. Alkanna tinctoria is used as a pigment in the coloration of food in Europe and North America. It also contributes to the vascularisation of the wound, shortening the healing period.

### Manufacturing Process:

Manufacturing process of the invention starts with melting the olive in until it reaches the smoking point. Smoking point of olive oil is average 180 - 220°C. The smoking point has a range, because it changes due to the olive oil's type, purity, acidity, and concentration.

By adding beeswax into the molten olive oil, it is provided that beeswax fully melts in the olive oil. After heating the mixture at the smoking point of the olive oil for 3-8 minutes, alkanna tinctoria is added to this mixture and again is heated at the smoking point of the olive oil for 5-8 minutes. Smoking point of the olive oil is taken as a basis for smoking point.

With filtering the mixture that is obtained at the end of this process through a fine-mesh filter that would not pass the particles, it is provided that alkanna tinctoria residues and granules are separated.

Before being applied on the burn wound, the mixture is sterilized by being placed in 100cc disposable glass amber bottles for each dressing. Before being placed in autoclave for sterilization, especially the cap area is wrapped with aluminum foil. Autoclave does the sterilization process at 121°C. To prevent the physical properties of the cap areas of the glass amber bottles due to high temperatures, wrapping with aluminium foil is necessary. Sterilization is done in the autoclave at 121°C under 1,5 atmospheric pressure.

After sterilization, bottles are left to coold down at room temperature. After cooling down, just before applying a mixture that could solidify a little on the wound, it is mixed properly shaked and rinsed for once. In this invention, disposable and low-cost abeslang is used as the sterile apparatus. Finally it is stored at +4°C.

### Application of Wound Care Composition on the Wounded Tissue:

Wound care composition is made into a dressing material after a specific period. After opening the wounds of patients, the wounds are first cleaned by irrigation with 0.09 NaCl and 0.010 savlon. The prepared sterile wound care composition is applied on the wound in a way to create a lock on the sterile sponge that is made proportionally with the wound size, followed by the closure of wound with the sterile sponge or pad (vary on the size of the wound surface). With the term "creating a lock" it is meant that, for the prepared wound care composition to be able to be applied on the wound, it has to be applied on a sponge or pad that could cover the wound completely. This lock application includes the wound care composition's pouring on the sponge or pad and the kneading manually in the hands of the wound care personnel. The purpose of kneading is to provide the total absorption and distribution of the wound care composition by sponge of pad.

Sterile sponge or pad that was put on the wound is fixated with gauze. In order to prevent the gauze from sliding and opening the wound area together with the sterile sponge/pad that covers the wound surface, it is fixed with an adhesive that would not traumatize the skin while being removed. This adhesive material can be betafix. Wound care composition is applied on the wounded tissue once a day.

Briefly, the method of preparing wound care composition comprises the operation steps of:
- heating medical olive oil up to its smoking point,
- melting beeswax by means of adding the beeswax into the olive oil that reached the smoking point,
- after melting of the beeswax completely in the olive oil that is found at the smoking point, heating the olive oil at the smoking point for about 3-8 minutes,
- after adding alkanna tinctoria to the obtained mixture, stirring at the smoking point of olive oil for about 5-8 minutes,
- while the obtained mixture is still warm, passing it through a filter with ultra fine pores so as to separate the alkanna tinctoria particles found therein,
- adding the liquid-consistency composition into 100 cc glass amber bottles,
- wrapping especially the lid parts of amber bottles with aluminium foil and placing into autoclave for sterilization,
- allowing the bottles sterilized in autoclave to cool down at room temperature,
- after cooled down at room temperature, thoroughly stirring and shaking the mixture once by a sterile tongue depressor just before applying onto the wound,
- storing the prepared mixture at +4 °C after it is cooled down.

## Claims

1. A wound care composition consisting of 2.78 % beeswax, 92.59 % medical olive oil with an acid ratio less than 0.8 %, and 4.63 % alkanna tinctoria by weight for use in a method of treating 2^{nd} degree burn wounds.

2. The wound care composition for use according to claim 1, wherein preparing composition comprising the operation steps of:
- heating medical olive oil up to its smoking point which is 180 - 220°C,
- melting beeswax by means of adding the beeswax into the olive oil that reached the smoking point,
- after melting of the beeswax completely in the olive oil, heating the olive oil at the smoking point for about 3-8 minutes,
- adding alkanna tinctoria into the prepared mixture,
- stirring the obtained composition,
- while the obtained mixture is still warm, passing it through a filter with ultra fine pores so as to separate the alkanna tinctoria particles found therein.

3. The wound care composition for use according to claim 3, wherein; comprising the operation steps of:
- adding the liquid-consistency composition into 100 cc glass amber bottles,
- placing amber bottles into autoclave,
- allowing the bottles sterilized in autoclave to cool down at room temperature,
- after cooled down at room temperature, thoroughly stirring and shaking the mixture once by a sterile apparatus just before applying onto the wound,
- storing the prepared mixture at +4 °C.

4. The wound care composition for use according to claim 4, wherein;
- especially the lid parts of amber bottle is wrapped with aluminum foil to prevent the physical properties of the lid parts of the glass amber bottles from being deteriorated due to high temperatures.

5. The wound care composition for use according to claim 3, wherein; the operation of melting beeswax in olive oil is performed at the smoking point of olive oil.

6. The wound care composition for use according to claim 3, wherein; after adding alkanna tinctoria to the prepared mixture, it is stirred at the smoking point of olive oil for about 5-8 minutes.

7. The wound care composition for use according to claim 4, wherein said sterile apparatus is a tongue depressor.

## Patentansprüche

1. Wundversorgungszusammensetzung, bestehend aus 2,78 Gew.-% Bienenwachs, 92,59 Gew.-% medizinischem Olivenöl mit einem Säuregehalt von weniger als 0,8 %, und 4,63 Gew.-% Schminkwurz zur Verwendung in einem Verfahren zum Behandeln von Verbrennungswunden 2. Grades.

2. Wundversorgungszusammensetzung zur Verwendung nach Anspruch 1, wobei das Herstellen der Zusammensetzung die folgenden Vorgangsschritte umfasst:
- Erhitzen von medizinischem Olivenöl bis zu seinem Rauchpunkt von 180 bis 220 °C,
- Schmelzen von Bienenwachs durch Hinzufügen des Bienenwachses zu dem Olivenöl, das den Rauchpunkt erreicht hat,
- nach dem vollständigen Schmelzen des Bienenwachses in dem Olivenöl Erhitzen des Olivenöls bei dem Rauchpunkt für etwa 3 bis 8 Minuten,
- Hinzufügen von Schminkwurz zu der hergestellten Mischung,
- Rühren der erhaltenen Zusammensetzung,
- während die erhaltene Mischung noch warm ist, Geben derselben durch einen Filter mit ultrafeinen Poren, um die darin gefundenen Schminkwurzpartikel abzutrennen.

3. Wundversorgungszusammensetzung zur Verwendung nach Anspruch 3, wobei sie die folgenden Vorgangsschritte umfasst:
- Hinzufügen der Zusammensetzung in flüssiger Konsistenz in bernsteinfarbene Glasflaschen von 100 cc,
- Platzieren der bernsteinfarbenen Flaschen in einem Autoklaven,
- Zulassen, dass die Flaschen in dem Autoklaven sterilisiert werden, um sie auf Raumtemperatur abzukühlen,
- nach dem Abkühlen auf Raumtemperatur gründliches Rühren und Schütteln der Mischung einmal durch eine sterile Vorrichtung direkt vor dem Auftragen auf die Wunde,
- Lagern der hergestellten Mischung bei +4 °C.

4. Wundversorgungszusammensetzung zur Verwendung nach Anspruch 4, wobei:
- insbesondere die Deckelteile der bernsteinfarbenen Flasche mit Aluminiumfolie umwickelt ist, um zu verhindern, dass sich die physikalischen Eigenschaften der Deckelteile der bernsteinfarbenen Glasflaschen aufgrund hoher Temperaturen verschlechtern.

5. Wundversorgungszusammensetzung zur Verwendung nach Anspruch 3, wobei der Vorgang des Schmelzens von Bienenwachs in Olivenöl an dem Rauchpunkt von Olivenöl durchgeführt wird.

6. Wundversorgungszusammensetzung zur Verwendung nach Anspruch 3, wobei nach dem Hinzufügen von Schminkwurz zu der hergestellten Mischung dieselbe bei dem Rauchpunkt von Olivenöl für etwa 5 bis 8 Minuten gerührt wird.

7. Wundversorgungszusammensetzung zur Verwendung nach Anspruch 4, wobei die sterile Vorrichtung ein Zungenspatel ist.

## Revendications

1. Composition de soin des plaies constituée de 2,78 % de cire d'abeille, de 92,59 % d'huile d'olive médicale avec un taux d'acidité inférieur à 0,8 %, et de 4,63 % d'alkanna tinctoria en poids destinée à être utilisée dans un procédé de traitement de lésions de brûlures au 2^{ème} degré.

2. Composition de soin des plaies destinée à être utilisée selon la revendication 1, dans laquelle la préparation de la composition comprend les étapes de mise en œuvre suivantes :
- chauffage de l'huile d'olive médicale jusqu'à son point de fumée qui est de 180 à 220 °C,
- fonte de la cire d'abeille en ajoutant la cire d'abeille dans l'huile d'olive qui a atteint son point de fumée,
- après la fonte complète de la cire d'abeille dans l'huile d'olive, chauffage de l'huile d'olive au point de fumée pendant environ 3 à 8 minutes,
- ajout d'alkanna tinctoria dans le mélange préparé,
- agitation de la composition obtenue,
- pendant que le mélange obtenu est encore chaud, passage de celui-ci à travers un filtre avec des pores ultrafins de manière à séparer les particules d'alkanna tinctoria qui s'y trouvent.

3. Composition de soin des plaies destinée à être utilisée selon la revendication 3, dans laquelle ; comprenant les étapes suivantes :
- ajout de la composition de consistance liquide dans des bouteilles en verre de couleur ambre de 100 cc,
- mise en place des bouteilles couleur ambre dans un autoclave,
- le fait de laisser les bouteilles stérilisées dans l'autoclave refroidir à température ambiante,
- après refroidissement à température ambiante, agitation approfondie et brassage du mélange une fois par un appareil stérile juste avant l'application sur la plaie,
- stockage du mélange préparé à +4 °C.

4. Composition de soin des plaies destinée à être utilisée selon la revendication 4, dans laquelle ;
- les parties de couvercle en particulier de la bouteille de couleur ambre sont enveloppées avec du papier d'aluminium pour empêcher que les propriétés physiques des parties de couvercle des bouteilles en verre de couleur ambre ne se détériorent en raison des températures élevées.

5. Composition de soin des plaies destinée à être utilisée selon la revendication 3, dans laquelle ; l'opération de fonte de la cire d'abeille dans l'huile d'olive est réalisée au point de fumée de l'huile d'olive.

6. Composition de soin des plaies destinée à être utilisée selon la revendication 3, dans laquelle ; après l'ajout d'alkanna tinctoria au mélange préparé, il est agité au point de fumée de l'huile d'olive pendant environ 5 à 8 minutes.

7. Composition de soin des plaies destinée à être utilisée selon la revendication 4, dans laquelle ledit appareil stérile est un abaisse-langue.
